# EUROPEAN PATENT SPECIFICATION

(11) **EP 0 780 106 B1**
(45) Date of publication and mention of the grant of the patent: **24.09.2003**
(21) Application number: 96120151.4
(22) Date of filing: 16.12.1996
(51) Int. Cl.: A61F 2/36

(54) **Femoral component of a hip joint endoprosthesis**
Femurteil einer Hüftgelenkendoprothese
Partie fémorale d'une endoprothèse de l'articulation de la hanche

(30) Priority: 20.12.1995 DE 19547638
(43) Date of publication of application: 25.06.1997
(73) Proprietor: Knipprath, Johannes, Dr., 14197 Berlin (DE); ENDOPLANT GmbH, 45768 Marl (DE)
(72) Inventor: Höppner, Dirk, 24248 Mönkeberg (DE); Knipprath, Johannes, Dr., 12157 Berlin (DE)
(74) Representative: Eisenführ, Speiser & Partner

(56) References cited:
- EP-A- 0 093 869
- EP-A- 0 112 435
- WO-A-87/00033
- DE-A- 3 829 361
- FR-A- 2 637 493
- FR-A- 2 693 367
- GB-A- 2 096 003
- GB-A- 2 203 943
- US-A- 4 068 324

## Description

The invention concerns a femoral component of a hip joint endoprosthesis for cementless and cemented implantation, with a shaft whose cross-section increases gradually from the distal towards the proximal end of the shaft, the increase of the cross-section in the ventrodorsal direction at the proximal shaft portion and towards the proximal end of the shaft taking place in several steps.

Such a femoral component of a hip joint endoprosthesis is known from FR-A-2693367. The proximal shaft portion of this known femoral component provides a substantially triangular cross-section the width of which increases step-wise from the medial to the lateral side of the shaft. As this triangular cross-section does not conform to the shape of the femur lumen, any cementless implantation will require additional resection of bone material in the femur lumen resulting in undue loading of the adjacent portions of the weakened femur.

DE-A-38 29 361 discloses a femoral component designed to be cemented in place which comprises a shaft whose cross-section is longitudinally adapted to the bone lumen of the natural femur, and which has a flange-type supporting collar at the proximal end of the shaft that rests against the resection edge of the natural femur when the femoral component is cemented into the femur.

EP 0 112 435 concerns a femoral component which increases in cross-section from the distal to the proximal end of the stem, the increases occurring in steps in the distal part of the stem.

Since bone cement penetrates non-uniformly under the supporting collar during the cementing process, direct contact between the supporting collar and the natural femur cannot be reliably prevented, and there is also a risk that in this area thin sections of bone cement may splinter under loading. Furthermore, because of the different material properties, undesired relative movement between the metallic supporting collar and the natural femur can also not be reliably excluded, as a result of which the bone tissue will undergo undesired alteration. In addition, as a rule the supporting collar only makes point contact when resting against the natural femur - sometimes via a layer of bone cement - since the final position of the femoral component and hence that of the supporting collar depends on the course and shape of the bone lumen and cannot be precisely predicted when the resection cut is made. Thus, disadvantages cannot be excluded so far as the transfer of vertical loading via a support collar to the natural femur is concerned.

It is therefore the object of the present invention to realize a femoral component of the type described above, which has no supporting collar involved in the transfer of vertical loading and has load transfer in a form-locking way over relatively large support surfaces.

In accordance with this invention there is provided a femoral component of a hip joint endoprosthesis for cementless and cemented implantation respectively, with a shaft whose cross-section increases gradually from the distal towards the proximal end of the shaft, the increase of the cross-section in the ventrodorsal direction at the proximal shaft portion and towards the proximal end of the shaft taking place in several steps, characterized in that the cross-section is extended in the mediolateral direction in the proximal portion of the shaft to form a substantially oval shape in cross-section and that the mediolateral width of the steps increases from the distal step edge towards the proximal end of the shaft, at least one of the several steps being located in the proximal portion of the shaft, the ventrodorsal width of the proximal shaft portion increasing in several steps in the mediolateral and the lateromedial direction.

The advantages of the invention consist particularly in the fact that in the proximal portion of the shaft, the increase of cross-section - to adapt to the lumen of the natural femur - takes place in several steps that begin a certain distance away from the distal end of the shaft with a smaller mediolateral width, and whose mdiolateral width increases gradually towards the proximal end of the shaft in correspondence with the cross-section of the shaft. Since, when the femoral component according to the invention is to be implanted, the bone lumen is shaped beforehand using a tool in the dorsal and ventral areas to form stepped recesses of corresponding shape, the step edges of the prosthesis together with the corresponding ones of the bone lumen form a form-locking fit which ensures both primary and long-term stability and also makes for vertical load transfer to the bone over a relatively large area. The conforming step-shaped recesses in the internal wall of the femur are produced using a shaping tool adapted to the femoral component, and their volume is then filled completely by implantation of a uniform layer of cement and the femoral component itself. Alternatively, it is also possible to implant the prosthesis according to the invention without any cement. The desired shaping of the step-like recesses to conform with the stepped projections of the artificial femoral component can be made simply with a corresponding tool, a rasp or similar, since the working movement of the tool takes place only in the axial direction and the prosthesis is then inserted into the prepared bone lumen with a movement of the same kind. Any undefined movement of the prosthetic femoral component during insertion is avoided.

It is particularly preferable to position at least one step both on the ventral side and on the dorsal side of the proximal portion of the shaft, such that support is achieved symmetrically and in a form-locking way on both sides of the femoral component. The step begins a certain distance away from the distal end with a smaller width, and becomes wider upwards towards the proximal end of the shaft.

In accordance with a preferred embodiment of the invention, on the ventral and dorsal sides of the shaft there are in each case several steps towards the proximal end of the shaft, arranged above one another like a staircase. The edge of the next-higher step is preferably offset in each case some distance away from the edge of the step under it, so that there is also some lateral or medial stepping whereby the whole cross-section of the shaft increases towards the proximal end on all sides and therefore approximates to the shape of the bone lumen. This makes for more reliable and firmer seating of the prosthesis in the bone lumen.

In accordance with a preferred embodiment of the invention, the edge height of the individual steps is constant in each case and the step surfaces are flat and preferably parallel to one another. This design feature ensures a high strength and favourable load transfer characteristics, with advantageous hardening.

Alternatively, the edge height of the steps can gradually increase in the proximal direction, which makes for additional bone compression when the prosthesis is inserted.

For preference, a flat basal surface is formed on the ventral and dorsal sides of the proximal portion of the shaft, on which the steps - staggered with respect to one another in the longitudinal direction - are formed on both the dorsal and the ventral sides of the cross-section. The ventral and dorsal basal surfaces can be parallel.

At the proximal end of the shaft there is a conical boss to enable the attachment of a prosthesis head by means of a push-on joint. Alternatively, at the proximal end of the shaft there can be a prosthesis neck with a prosthesis head formed on it. In addition, at the proximal end of the shaft a lateral projection can also be formed, which increases the primary stability immediately after implantation of the prosthesis component, especially against rotational loads.

For preference, the cross-section of the shaft becomes circular towards the distal end, and along the distal portion of the shaft a wedge surface is formed laterally, which reduces the shaft cross-section towards the distal end to circular sections flat on one side.

In accordance with a preferred embodiment of the invention, the distal shaft portion of the prosthesis is bent towards the ventral direction by a specified angle α, preferably in the range 3° to 50°. This makes for better adaptation of the prosthesis shaft to the course of the bone lumen, so simplifying the implantation of the prosthesis.

Further advantageous features of the invention are characterized in the dependent claims.

In what follows, an embodiment of the invention will be explained with reference to the drawings, which show:
- Fig. 1:: A side view of the femoral component, seen from the ventral side.
- Fig. 2:: A cross-section along the line A-A in Fig. 1, through the proximal portion of the shaft.
- Fig. 3:: A lateral view of the femoral component.
- Fig. 4:: A medial view of the femoral component, and
- Fig. 5:: A perspective view of the femoral component.

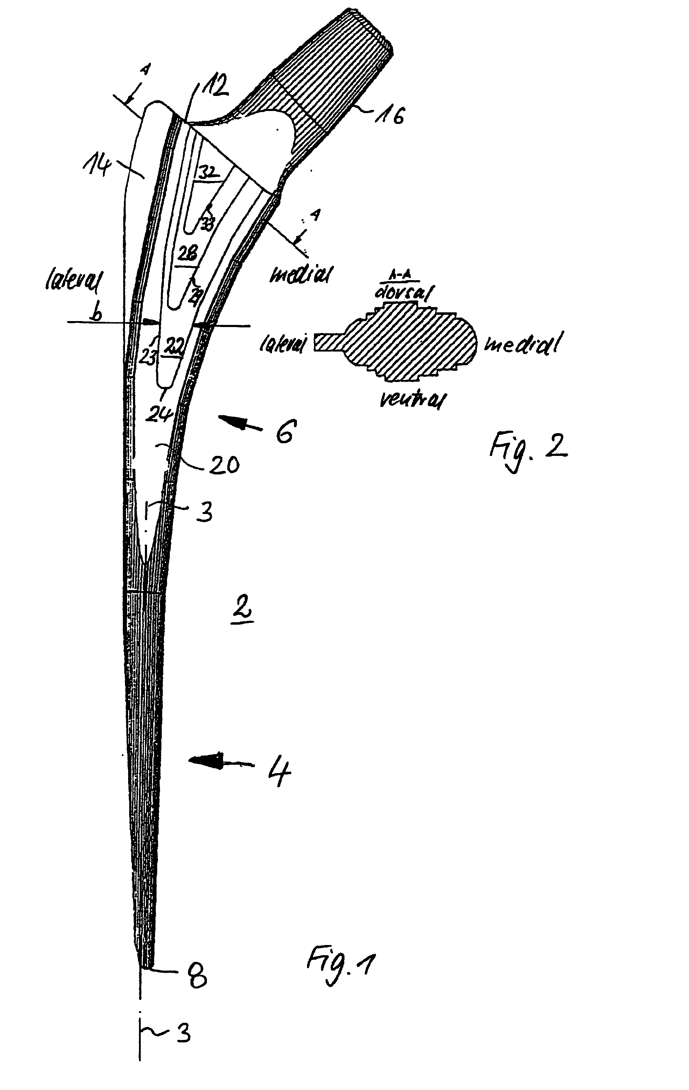

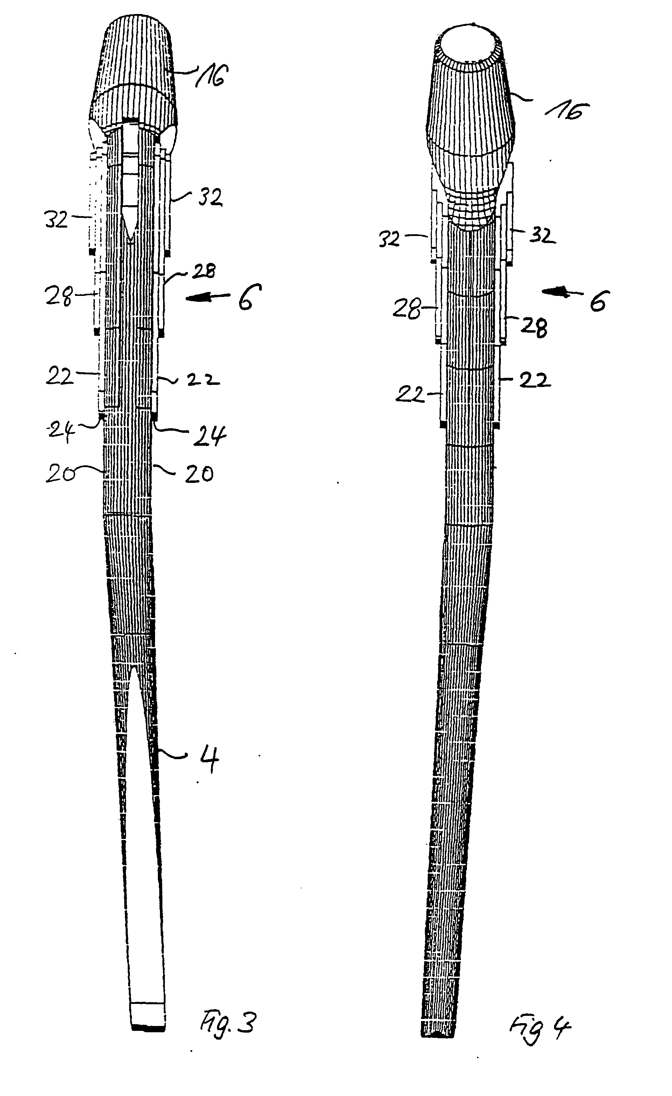

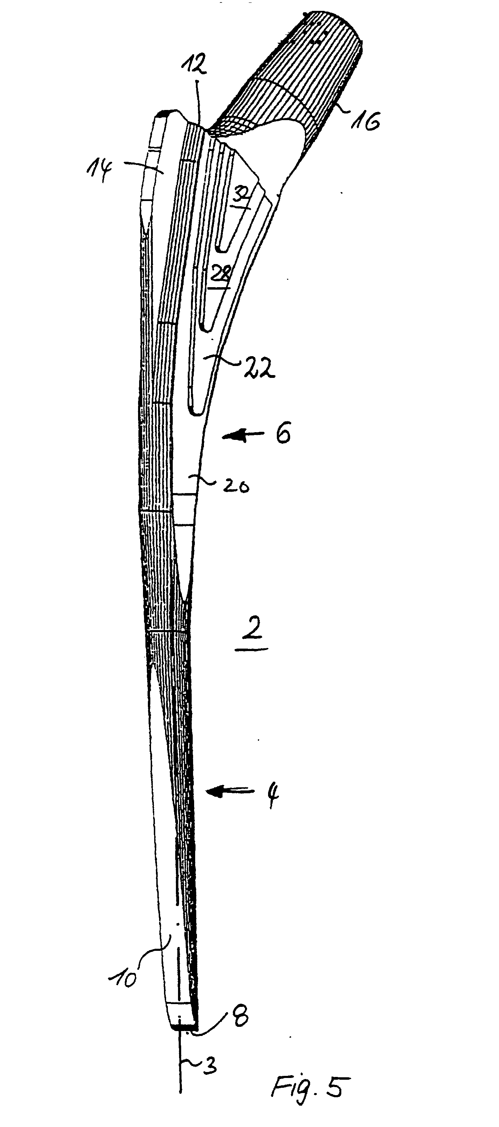

Figs. 1 to 5 show various views of a femoral component of a hip joint endoprosthesis. The femoral component comprises a shaft 2 whose cross-section increases gradually from the distal end 8 of the shaft towards the proximal end 12 of the shaft. Over its entire length, the cross-section of the shaft 2 is adapted to the bone lumen of the natural femur bone in man. Along the distal portion 4 of the shaft, its cross-section is almost circular, while along the proximal portion 6 the cross-section assumes an oval shape extended in the mediolateral direction, such that it increases more rapidly in the mediolateral direction than in the ventrodorsal direction.

At the proximal end 12 of the shaft 2 there is no supporting collar, but there is a lateral projection which increases the rotational stability, especially shortly after the implantation process.

At the proximal end 12 of the shaft a conical boss 16 is formed, inclined with respect to the longitudinal axis 3 of the shaft by the so-termed CCD angle. The conical boss 16 serves to allow attachment of a prosthesis head (not shown) with a corresponding conical socket, enabling the latter to be pushed on and attached firmly to the femoral component by adhesive bonding. Alternatively, it is also possible to form a prosthesis head with a neck in one piece at the proximal end of the shaft.

The distal portion 4 of the shaft - over a certain length - is offset or bent towards the ventral direction, to achieve better adaptation to the bone lumen of the human femur and thereby enable simpler implantation. Further, the distal portion 4 of the shaft has a wedge-shaped surface 10 formed laterally towards the distal end 8 of the shaft, which reduces the circular cross-section towards the bottom to circular sections flat on one side.

Along the proximal portion of the shaft 2 flat basal surfaces 20 are formed on the ventral and dorsal sides, and towards the proximal end 12 of the shaft the shaft cross-section increases in the ventrodorsal direction in several steps 22, 28, 32, which begin at a certain level with a distally facing step edge 24, 30, 34 and then rise towards the proximal end 12 of the shaft with increasing mediolateral width. At least one step 22 is located on both the ventral and dorsal sides of the shaft 2. However, in the embodiment illustrated there are several steps 22, 28, 32 on both the ventral and dorsal sides, staggered with respect to one another towards the proximal end of the shaft and whose step edges are each some distance away from the edge of the step below, so forming a stepped increase of the shaft cross-section. All the steps extend up to the proximal end 12 of the shaft, the first step 22 extending most widely in the distal direction, with the largest mediolateral width. The second step has a smaller distal extension and its mediolateral width is smaller than that of the first step. The third step 32 extends still less in the distal direction and its mediolateral width is smaller than that of the previous, second step 28. In the embodiment illustrated, the steps have a constant edge height all round and their surfaces are flat. As can be seen particularly in Figs. 3 and 4, the step surfaces are thus parallel to one another. The stepping on the ventral side is a mirror-image of that on the dorsal side of the shaft 2.

## Claims

1. Femoral component of a hip joint endoprosthesis for cementless and cemented implantation respectively, with a shaft whose cross-section increases gradually from the distal towards the proximal end of the shaft, the increase of the cross-section in the ventrodorsal direction at the proximal shaft portion and towards the proximal end (12) of the shaft taking place in several steps (22, 28, 32),
**characterized in that** the cross-section is extended in the mediolateral direction in the proximal portion of the shaft to form a substantially oval shape in cross-section and that the mediolateral width of the steps increases from the distal step edge (24) towards the proximal end (12) of the shaft, the distal end of at least one of the several steps being located in the proximal portion of the shaft, the ventrodorsal width of the proximal shaft portion increasing in several steps in the mediolateral and the lateromedial direction.

2. Femoral component according to claim 1,
**characterized in that** there is at least one step (22) both on the ventral and on the dorsal side of the shaft (2).

3. Femoral component according to claims 1 or 2,
**characterized in that** both on the ventral and on the dorsal side of the shaft (2) several steps (22, 28, 32) are arranged over and staggered with respect to one another towards the proximal end (12) of the shaft, and the edge (29) of each next-higher step (28) is some distance away from the edge (23) of the step (22) below it.

4. Femoral component according to any of the preceding claims,
**characterized in that** the edge heights of the individual steps (22, 28, 32) are the same for each step.

5. Femoral component according to any of the preceding claims,
**characterized in that** the edge height of all the steps (22, 28, 32) has the same value.

6. Femoral component according to any of claims 1 to 3,
**characterized in that** the edge height of individual steps increases gradually towards the proximal direction.

7. Femoral component according to any of the preceding claims,
**characterized in that** the surfaces of the steps (22, 28, 32) are flat.

8. Femoral component according to any of the preceding claims,
**characterized in that** the surfaces of the ventral steps (22, 28, 32) are parallel to the surfaces of the dorsal steps (22, 28, 32).

9. Femoral component according to any of the preceding claims,
**characterized in that** in the proximal area of the shaft (2) on both the ventral and the dorsal sides, a flat basal surface (20) is formed, on which the first step (22) is located.

10. Femoral component according to claim 9,
**characterized in that** the ventral and dorsal basal surfaces (20) are parallel to one another.

11. Femoral component according to any of the preceding claims,
**characterized in that** at the proximal end (12) of the shaft (23) a conical plug (16) is provided for the attachment of a prosthesis head.

12. Femoral component according to any of claims 1 to 10,
**characterized in that** at the proximal end (12) of the shaft (2), a prosthesis neck with a prosthesis head formed on it is located.

13. Femoral component according to any of the preceding claims,
**characterized in that** on the proximal portion of the shaft (2) a lateral projection (14) is formed.

14. Femoral component according to any of the preceding claims,
**characterized in that** the cross-section of the shaft (2) changes to a circular section towards the distal end.

15. Femoral component according to any of the preceding claims,
**characterized in that** along the distal portion (4) of the shaft (2) a lateral wedge surface (10) is formed.

16. Femoral component according to any of the preceding claims,
**characterized in that** a distal portion (4) of the shaft is bent towards the ventral direction.

## Patentansprüche

1. Femorale Komponente einer Hüftgelenkendoprothese zur entsprechenden zementlosen und zementierten Implantation, mit einem Schaft, dessen Querschnitt fortschreitend von dem distalen in Richtung auf das proximale Ende zunimmt, wobei diese Querschnittszunahme in der ventro-dorsalen Richtung an dem proximalen Schaftabschnitt in Richtung auf das proximale Ende (12) des Schaftes in mehreren Stufen (22, 28, 32) erfolgt,
**dadurch gekennzeichnet, dass** der Querschnitt im proximalen Schaftabschnitt in der medio-lateralen Richtung gestreckt ist, um im Querschnitt eine im Wesentlichen ovale Form auszubilden, und dass die medio-laterale Breite der Stufen von der distalen Stufenkante (24) in Richtung auf das proximale Ende (12) des Schaftes zunimmt, wobei das distale Ende von wenigstens einer der mehreren Stufen in dem proximalen Schaftabschnitt angeordnet ist und die ventro-dorsale Breite des proximalen Schaftabschnittes in mehreren Stufen in der medio-lateralen und der latero-medialen Richtung zunimmt.

2. Femorale Komponente nach Anspruch 1,
**dadurch gekennzeichnet, dass** auf der ventralen und auf der dorsalen Seite des Schaftes (2) wenigstens je eine Stufe (22) vorhanden ist.

3. Femorale Komponente nach Anspruch 1 oder 2,
**dadurch gekennzeichnet, dass** auf der ventralen und auf der dorsalen Seite des Schaftes (2) jeweils mehrere Stufen (22, 28, 32) zum proximalen Schaftende (12) hin versetzt aufeinander angeordnet sind, und dass die Kante (29) der jeweils nächst-höheren Stufe (28) in einem gewissen Abstand zu der Kante (23) der darunterliegenden Stufe (22) angeordnet ist.

4. Femorale Komponente nach einem der vorhergehenden Ansprüche,
**dadurch gekennzeichnet, dass** die Kantenhöhen der einzelnen Stufen (22, 28, 32) pro Stufe gleich ist.

5. Femorale Komponente nach einem der vorhergehenden Ansprüche,
**dadurch gekennzeichnet, dass** die Kantenhöhe aller Stufen (22, 28, 32) den gleichen Wert aufweist.

6. Femorale Komponente nach einem der Ansprüche 1 bis 3,
**dadurch gekennzeichnet, dass** die Kantenhöhe der einzelnen Stufen in Richtung der proximalen Richtung fortlaufend zunimmt.

7. Femorale Komponente nach einem der vorhergehenden Ansprüche,
**dadurch gekennzeichnet, dass** die Oberflächen der Stufen (22, 28, 32) eben sind.

8. Femorale Komponente nach einem der vorhergehenden Ansprüche,
**dadurch gekennzeichnet, dass** die Oberflächen der ventralen Stufen (22, 28, 32) parallel zu den Oberflächen der dorsalen Stufen (22, 28, 32) sind.

9. Femorale Komponente nach einem der vorhergehenden Ansprüche,
**dadurch gekennzeichnet, dass** in dem proximalen Bereich des Schaftes (2) sowohl auf der ventralen als auch auf der dorsalen Seite eine ebene, Grundfläche (20) ausgebildet ist, auf der die erste Stufe (22) angeordnet ist.

10. Femorale Komponente nach Anspruch 9,
**dadurch gekennzeichnet, dass** die ventralen und dorsalen Grundflächen (20) parallel zueinander sind.

11. Femorale Komponente nach einem der vorhergehenden Ansprüche,
**dadurch gekennzeichnet, dass** an dem proximalen Ende (12) des Schaftes (23) ein Außenkonus (16) zum Befestigen eines Prothesenkopfes angeordnet ist.

12. Femorale Komponente nach einem der Ansprüche 1-10,
**dadurch gekennzeichnet, dass** an dem proximalen Ende (12) des Schaftes (2) ein Prothesenhals mit einem daran ausgeformten Prothesenkopf angeordnet ist.

13. Femorale Komponente nach einem der vorhergehenden Ansprüche,
**dadurch gekennzeichnet, dass** auf dem proximalen Abschnitt des Schaftes (2) ein lateraler Vorsprung (14) ausgebildet ist.

14. Femorale Komponente nach einem der vorhergehenden Ansprüche,
**dadurch gekennzeichnet, dass** der Querschnitt des Schaftes (2) sich in Richtung des distalen Endes in einen kreisförmigen Querschnitt ändert.

15. Femorale Komponente nach einem der vorhergehenden Ansprüche,
**dadurch gekennzeichnet, dass** entlang des distalen Abschnitts (4) des Schaftes (2) eine laterale Keilfläche (10) ausgebildet ist.

16. Femorale Komponente nach einem der vorhergehenden Ansprüche,
**dadurch gekennzeichnet, dass** ein distaler Abschnitt (4) des Schaftes nach ventral abgekröpft ist.

## Revendications

1. Composant fémoral d'une endoprothèse d'articulation coxo-fémorale pour implantation respectivement avec ciment et sans ciment, avec une broche dont la section transversale augmente graduellement de l'extrémité distale vers l'extrémité proximale de la broche, l'accroissement de la section transversale dans la direction ventro-dorsale sur la partie de la broche proximale et vers l'extrémité proximale (12) de la broche se faisant en plusieurs degrés (22, 28, 32),
**caractérisé en ce que** la section transversale est prolongée dans la direction médiolatérale dans la partie proximale de la broche afin de prendre une forme sensiblement ovalisée dans la section transversale et que la largeur médiolatérale des degrés augmente depuis le bord du degré distal (24) vers l'extrémité proximale (12) de la broche, l'extrémité distale d'au moins un des nombreux degrés étant située dans la partie proximale de la broche, la largeur ventro-dorsale de la partie de la broche proximale augmentant en plusieurs degrés dans les directions médiolatérale et latéromédiane.

2. Composant fémoral selon la revendication 1, **caractérisé en ce qu'**il y a au moins un degré (22) tant sur le côté ventral que sur le côté dorsal de la broche (2).

3. Composant fémoral selon la revendication 1 ou 2, **caractérisé en ce que**, tant sur le côté ventral que sur le côté dorsal de la broche (2), plusieurs degrés (22, 28, 32) sont disposés au-dessus et échelonnés l'un vis-à-vis de l'autre en direction de l'extrémité proximale (12) de la broche, et le bord (29) qui fait suite à chaque degré placé plus haut (28) est à une certaine distance du bord (23) du degré (22) en dessous de celui-ci.

4. Composant fémoral selon l'une quelconque des revendications précédentes, **caractérisé en ce que** les hauteurs des bords des différents degrés (22, 28, 32) sont identiques pour chaque degré.

5. Composant fémoral selon l'une quelconque des revendications précédentes, **caractérisé en ce que** la hauteur du bord de tous les degrés (22, 28, 32) a la même valeur.

6. Composant fémoral selon une des revendications 1 à 3, **caractérisé en ce que** la hauteur du bord des différents degrés augmente graduellement vers la direction proximale.

7. Composant fémoral selon l'une quelconque des revendications précédentes, **caractérisé en ce que** les surfaces des degrés (22, 28, 32) sont plates.

8. Composant fémoral selon l'une quelconque des revendications précédentes, **caractérisé en ce que** les surfaces des degrés ventraux (22, 28, 32) sont parallèles aux surfaces des degrés dorsaux (22, 28, 32).

9. Composant fémoral selon l'une quelconque des revendications précédentes, **caractérisé en ce que** la zone proximale de la broche (2), tant sur les côtés ventraux que les côtés dorsaux, un intrados plat (20) est formé sur lequel est situé le premier degré (22).

10. Composant fémoral selon la revendication 9, **caractérisé en ce que** les intrados ventral et dorsal (20) sont parallèles l'un par rapport à l'autre.

11. Composant fémoral selon l'une quelconque des revendications précédentes, **caractérisé en ce qu'**à l'extrémité proximale (12) de la broche (23) une prise conique (16) est prévue pour la fixation d'une tête de prothèse.

12. Composant fémoral selon l'une des revendications 1 à 10, **caractérisé en ce qu'**à l'extrémité proximale (12) de la broche (2) une gorge de prothèse sur laquelle est formée une tête de prothèse est située.

13. Composant fémoral selon l'une quelconque des revendications précédentes, **caractérisé en ce que** sur la partie proximale de la broche (2), une expansion latérale (14) est formée.

14. Composant fémoral selon l'une quelconque des revendications précédentes, **caractérisé en ce que** la section transversale de la broche (2) se transforme en une section circulaire vers l'extrémité distale.

15. Composant fémoral selon l'une quelconque des revendications précédentes, **caractérisé en ce que** le long de la partie distale (4) de la broche (2), une surface inclinée (10) est formée.

16. Composant fémoral selon l'une quelconque des revendications précédentes, **caractérisé en ce qu'**une partie distale (4) de la broche est inclinée vers la direction ventrale.
